# EUROPÄISCHE PATENTSCHRIFT

(11) **EP 1 189 522 B1**
(45) Veröffentlichungstag und Bekanntmachung des Hinweises auf die Patenterteilung: **21.09.2005**
(21) Anmeldenummer: 00938794.5
(22) Anmeldetag: 16.06.2000
(51) Int. Cl.: A23L 1/30

(54) **VERWENDUNG VON NANOSKALIGEN STEROLEN UND STEROLESTERN**
USE OF NANOSCALE STEROLS AND STEROL ESTERS
UTILISATION DE STEROLS ET DE STEROLS ESTERIFIES DE L'ORDRE DU NANOMETRE

(30) Priorität: 25.06.1999 US 141154 P
(43) Veröffentlichungstag der Anmeldung: 27.03.2002
(73) Patentinhaber: Cognis IP Management GmbH, 40589 Düsseldorf (DE)
(72) Erfinder: KROPF, Christian, D-40597 Düsseldorf (DE); FABRY, Bernd, D-41352 Korschenbroich (DE); BIERMANN, Manfred, Cincinnati, OH 45242 (US); DOLHAINE, Hans, D-41352 Glehn (DE); CHRISTOPHLIEMK, Peter, D-40595 Düsseldorf (DE); SCHRÖDER, Christine, D-40593 Düsseldorf (DE)
(86) Internationale Anmeldenummer: PCT/EP2000/005537
(87) Internationale Veröffentlichungsnummer: WO 2001/000046

(56) Entgegenhaltungen:
- EP-A- 0 087 993
- EP-A- 0 897 671
- WO-A-00/21490
- WO-A-99/59421
- WO-A-99/60869
- WO-A-99/63841
- "Micronization of organic solids by rapid expansion of supercritical solutions" STN CHEMICAL ABSTRAC, XP002126942
- "Effect of process parameters on particles obtained by rapid expansion of supercritical solutions" STN CHEMICAL ABSTRAC, XP002126943

## Beschreibung

Die Erfindung befindet sich auf dem Gebiet der Nanopartikel und betrifft die Verwendung von nanoskaligen Sterolen und Sterolestern als Lebensmittelzusatzstoffe.

### Stand der Technik

Sterole und Sterolester stellen wichtige Rohstoffe sowohl für die Kosmetik und Pharmazie als auch für die Nahrungsmittelindustrie dar. So gibt es beispielsweise Hinweise dafür, daß Sterole, insbesondere pflanzliche Vertreter ("Phytosterole"), in die Basalmembran der Haut eingebaut werden und über die Differenzierung der Hautzellen an die Hautoberfläche gelangen. Dies würde die pflegende und schützende Wirkung von Phytosterolen in der Hautkosmetik erklären. Die topische Anwendung von Sterolen führt auch zu einer erhöhten Hautfeuchtigkeit und einem gesteigerten Lipidgehalt. Hierdurch wird das Schuppungsverhalten der Haut verbessert und vorhandene Erytheme reduziert. Übersichten zu den Eigenschaften von Sterolen und Sterolestern in der Kosmetik finden sich beispielsweise von R.Wachter in **Parf.Kosm.** **75****, 755 (1994)** bzw. **Cosm.Toil.** **110****, 72 (1995).** Eine weitere wichtige Eigenschaft von Phytosterolen und hier vor allem von Phytosterolestem ist ihre hypocholesterinämische Wirkung, d.h. ihre Fähigkeit, bei oraler Aufnahme, z.B. als Zusatzstoff zu Margarine, den Cholesteringehalt im Blut signifikant zu reduzieren, die schon 1953 von Peterson et al. in **J.Nutrit.** **50****, 191 (1953)** beschrieben wurde. In die gleiche Richtung weisen auch die Patentschriften **US 3,089,939, US 3,203,862** sowie die deutsche Offenlegungsschrift **DE-OS 2035069** (Procter & Gamble). Die Wirkstoffe werden üblicherweise Brat-oder Speiseölen zugesetzt und dann über die Nahrung aufgenommen, wobei die Einsatzmengen jedoch in der Regel gering sind und üblicherweise unter 0,5 Gew.-% liegen, um zu verhindern, daß die Speiseöle eintrüben oder die Sterole bei Zusatz von Wasser ausgefällt werden. Die Einarbeitung von Sitostanolestem zur Verminderung des Blutcholesteringehaltes in Margarine, Butter, Mayonnaise, Salatsaucen und dergleichen wird in der internationalen Patentanmeldung **WO 92/19640** (Raision) vorgeschlagen. In diesem Zusammenhang sei femer auch auf die deutsche Patentanmeldung **DE-A1 19700796** (Henkel) und auf EP 0 897 671 (Unilever) verwiesen.

Die Wirkung der Sterole und Sterolester hängt stets mit der Geschwindigkeit zusammen, mit der die Verbindungen resorbiert werden. Hier besteht für die verfügbaren Stoffe des Stands der Technik noch ein erhebliches Verbesserungspotential. Die Aufgabe der vorliegenden Erfindung hat somit darin bestanden, die Aufnahme von Sterole und Sterolester bei oraler Aufnahme durch Bereitstellung neuer Anbietungsformen zu beschleunigen.

### Beschreibung der Erfindung

Gegenstand der Erfindung ist die Verwendung von nanoskaligen Sterolen und/oder Sterolestem, welche von einem toxikologisch umbedenklichen Schutzkolloid ummantelt vorliegen, mit Teilchendurchmessem im Bereich von 10 bis 300 nm als Lebensmittelzusatzstoffe sowie als Wirkstoffe zur Herstellung hypocholesterinämischer Mittel.

Überraschenderweise wurde gefunden, daß sich die Resorption und hypocholesterinämische Wirkung von Sterolen und Sterolestem, insbesondere solchen auf Basis pflanzlicher Rohstoffe, signifikant steigern läßt, wenn diese in Form von Nanoteilchen, d.h. Partikeln mit einem mittleren Durchmesser im Bereich von 10 bis 300 und vorzugsweise 50 bis 150 nm vorliegen. Dabei ist zwischen zwei Ausführungsformen zu unterscheiden, nämlich der direkten Einarbeitung der Nanopartikel in die Lebensmittel und die Verkapselung der Partikel zur separaten oralen Aufnahme. Die Erfindung schließt ferner die Erkenntnis ein, daß die nanoskaligen Sterole bzw. Sterolester eine verbesserte Löslichkeit bzw. Dispergierbarkeit aufweisen, so daß es nunmehr möglich ist auch größere Einsatzmengen beispielsweise in Speiseölen klar und dauerhaft einzuarbeiten.

### Sterole und Sterolester

Unter Sterolen (oder synonym Stenolen) sind tierische bzw. pflanzliche Steroide zu verstehen, die nur am C-3 eine Hydroxylgruppe, sonst aber keine funktionellen Gruppen tragen. In der Regel besitzen die Sterole 27 bis 30 Kohlenstoffatome und eine Doppelbindung in 5/6, gegebenenfalls 7/8, 8/9 oder anderen Positionen. Neben diesen ungesättigten Spezies kommen als Sterole auch die durch Härtung erhältlichen gesättigten Verbindungen in Frage, die als Stanole bezeichnet werden und von der vorliegenden Erfindung mitumschlossen werden. Ein Beispiel für ein geeignetes tierisches Sterol ist Cholesterol. Typische Beispiele für geeignete Phytosterole, welche aus anwendungstechnischen Gründen bevorzugt werden, sind beispielsweise Ergosterole, Campesterole, Stigmasterole, Brassicasterole sowie vorzugsweise Sitosterole bzw. Sitostanole und insbesondere β-Sitosterole bzw. β-Sitostanole. Neben den genannten Phytosterolen werden vorzugsweise deren Ester eingesetzt. Die Säurekomponente der Esters kann auf Carbonsäuren der Formel (I) zurückgehen,

**R**^{**1**}**CO-OH** (I)

in der R¹CO für einen aliphatischen, linearen oder verzweigten Acylrest mit 2 bis 22 Kohlenstoffatomen und 0 und/oder 1, 2 oder 3 Doppelbindungen steht. Typische Beispiele sind Essigsäure, Propionsäure, Buttersäure, Valeriansäure, Capronsäure, Caprylsäure, 2-Ethylhexansäure, Caprinsäure, Laurinsäure, Isotridecansäure, Myristinsäure, Palmitinsäure, Palmoleinsäure, Steacinsäure, Isostearinsäure, Ölsäure, Elaidinsäure, Petroselinsäure, Linolsäure, konjugierte Linolsäure (CLA), Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Reduktion von Aldehyden aus der Roelen'schen Oxosynthese oder als Monomerfraktion bei der Dimerisierung von ungesättigten Fettsäuren anfallen. Bevorzugt sind technische Fettsäuren mit 12 bis 18 Kohlenstoffatomen wie beispielsweise Kokos-, Palm-, Palmkern- oder Talgfettsäure. Besonders bevorzugt ist der Einsatz von Estern des β-Sitosterols bzw. β-Sitostanols mit Fettsäuren mit 12 bis 18 Kohlenstoffatomen. Diese Ester können sowohl durch direkte Veresterung der Phytostenole mit den Fettsäuren oder aber durch Umesterung mit Fettsäureniedrigalkylestern oder Triglyceriden in Gegenwart geeigneter Katalysatoren, wie z.B. Natriumethylat oder speziell auch Enzymen hergestellt werden [vgl. **EP-A2 0195311** (Yoshikawa)].

### Herstellung von Nanopartikeln

Ein solches Verfahren zur Herstellung von Nanoteilchen durch rasche Entspannung von überkritischen Lösungen **(Rapid Expansion of Supercritical Solutions RESS)** ist beispielsweise aus dem Aufsatz von S.Chihlar, M.Türk und K.Schaber in **Proceedings World Congress on Particle Technology 3, Brighton, 1998** bekannt. Um zu verhindern, daß die Nanoteilchen wieder zusammenbacken, empfiehlt es sich, die Nanopartikel entweder unmittelbar nach der Herstellung den Lebensmitteln zuzusetzen oder aber die Ausgangsstoffe in Gegenwart geeigneter, d.h. insbesondere toxikologisch unbedenklicher Schutzkolloide zu lösen und/oder die kritischen Lösungen in wäßrige und/oder alkoholische Lösungen der Schutzkolloide zu entspannen, welche ihrerseits wieder gelöste Emulgatoren und/oder Schutzkolloide enthalten können. Geeignete Schutzkolloide sind dabei z.B. Gelatine, Chitosan, Casein, Gummi arabicum, Lysalbinsäure, Stärke sowie Polymere, wie etwa Polyvinylalkohole, Polyvinylpyrrolidone Polyalkylenglycole und Polyacrylate. Die zu verwendenden nanoskaligen Sterole und/oder Sterolester sind also die, die von einem toxikologisch unbedenklichen Schutzkolloid ummantelt vorliegen. Vorzugsweise kommen hier Gelatine, Chitosan oder deren Abmischungen in Betracht. Üblicherweise werden die Schutzkolloide in Mengen von 0,1 bis 20, vorzugsweise 5 bis 15 Gew.-% - bezogen auf die Sterole bzw. Sterolester - eingesetzt.

Ein weiteres geeignetes Verfahren zur Herstellung der nanoskaligen Teilchen bietet die **Evaporationstechnik.** Hierbei werden die Ausgangsstoffe zunächst in einem geeigneten organischen Lösungsmittel (z.B. Alkane, pflanzliche Öle, Ether, Ester, Ketone, Acetale und dergleichen) gelöst. Anschließend werden die Lösungen derart in Wasser oder einem anderen Nicht-Lösungsmittel, gegebenenfalls in Gegenwart einer darin gelösten oberflächenaktiven Verbindung gegeben, daß es durch die Homogenisierung der beiden nicht miteinander mischbaren Lösungsmittel zu einer Ausfällung der Nanoteilchen kommt, wobei das organische Lösungsmittel vorzugsweise verdampft. Anstelle einer wäßrigen Lösung können auch O/W-Emulsionen bzw. O/W-Mikroemulsionen eingesetzt werden. Als oberflächenaktive Verbindungen können die bereits eingangs erläuterten Emulgatoren und Schutzkolloide verwendet werden. Eine weitere Möglichkeit zur Herstellung von Nanoteilchen besteht in dem sogenannten **GAS-Verfahren** (Gas Anti Solvent Recrystallization). Das Verfahren nutzt ein hochkomprimiertes Gas oder überkritisches Fluid (z.B. Kohlendioxid) als Nicht-Lösungsmittel zur Kristallisation von gelösten Stoffen. Die verdichtete Gasphase wird in die Primärlösung der Ausgangsstoffe eingeleitet und dort absorbiert, wodurch sich das Flüssigkeitsvolumen vergrößert, die Löslichkeit abnimmt und feinteilige Partikel ausgeschieden werden. Ähnlich geeignet ist das **PCA-Verfahren** (Precipitation with a Compressed Fluid Anti-Solvent). Hier wird die Primärlösung der Ausgangsstoffe in ein überkritisches Fluid eingeleitet, wobei sich feinstverteilte Tröpfchen bilden, in denen Diffusionsvorgänge ablaufen, so daß eine Ausfällung feinster Partikel erfolgt. Beim **PGSS-Verfahren** (Particles from Gas Saturated Solutions) werden die Ausgangsstoffe durch Aufpressen von Gas (z.B. Kohlendioxid oder Propan) aufgeschmolzen. Druck und Temperatur erreichen nahe- oder überkritische Bedingungen. Die Gasphase löst sich im Feststoff und bewirkt eine Absenkung der Schmelztemperatur, der Viskosität und der Oberflächenspannung. Bei der Expansion durch eine Düse kommt es durch Abkühlungseffekte zur Bildung feinster Teilchen.

### Gewerbliche Anwendbarkeit

Gegenüber Sterolen und Sterolestern des Stands der Technik bewirkt die besondere Feinteiligkeit der Partikel bei oraler Aufnahme eine raschere Resorption durch das Blutserum. Neben der in situ-Verkapselung der Nanopartikel ist es auch möglich, die Stoffe in üblichen Nahrungsmitteln zu lösen bzw. zu dispergieren, als da beispielsweise sind : Butter, Margarine, Diätnahrung, Fritieröle, Speiseöle, Mayonnaisen, Salatdressings, Kakaoprodukte, Wurst und dergleichen. Die Einsatzmenge der nanoskaligen Verbindungen liegt üblicherweise in der Größenordnung von 0,01 bis 5, vorzugsweise 0,1 bis 2 und insbesondere 0,5 bis 1 Gew.-% - bezogen auf die Mittel.

### Beispiele

**Herstellbeispiele.** Zur Herstellung der nanoskaligen Sterole und Sterolester **(Beispiele 1 bis 5)** wurde zunächst Kohlendioxid einem Reservoir mit einem konstanten Druck von 60 bar entnommen und über eine Kolonne mit einer Aktivkohle- und einer Molekularsieb-Packung gereinigt. Nach der Verflüssigung wurde das CO₂ mit Hilfe einer Diaphragma-Pumpe bei einer konstanten Fördermenge von 3,5 l/h auf den gewünschten überkritischen Druck p verdichtet. Anschließend wurde das Lösungsmittel in einem Vorheizer auf die erforderliche Temperatur T1 gebracht und in eine Extraktionskolonne (Stahl, 400 ml) geleitet, welche mit dem Sterol bzw. Sterolester beladen war. Die resultierende überkritische, d.h. fluide Mischung wurde über eine lasergezogene Düse (Länge 830 µm, Durchmesser 45 µm) bei einer Temperatur T2 in eine Plexiglas Expansionskammer versprüht, die eine 4 Gew.-%ige wäßrige Dispersion eines Schutzkolloids enthielt. Das fluide Medium verdampfte und zurück blieben die im Schutzkolloid eingeschlossenen, dispergierten Nanopartikel. Zur Herstellung der Nanoteilchen gemäß **Beispiel 6** wurde eine 1 Gew.-%ige Lösung von Phytosterol in Aceton unter starkem Rühren bei 40°C und einem verminderten Druck von 40 mbar in eine 4 Gew.-% wäßrige Dispersion einer Mischung von Gelatine und Chitosan getropft. Das verdampfende Lösungsmittel wurde in einer Kühlfalle kondensiert, während die Dispersion mit den Nanopartikeln zurückblieb. Die Verfahrensbedingungen und der mittlere Partikelgrößenbereich (Bsp. 1 bis 5 photometrisch nach der 3-WEM-Methode, Bsp.6 über Laser-Rückstreuung bestimmt) sind in der nachfolgenden Tabelle 1 angegeben.

**Tabelle 1**

| **Nanopartikel** | | | | | | | |
|---|---|---|---|---|---|---|---|
| **Bsp.** | **Sterol/Sterolester** | **Lsgm.** | **p** **bar** | **T1** **°C** | **T2** **°C** | **Schutzkolloid** | **PGB** **nm** |
| 1 | Phytosterol* | CO₂ | 200 | 80 | 175 | Gelatine | 60-135 |
| 2 | Phytosterol* | CO₂ | 180 | 70 | 160 | Gelatine | 75-125 |
| 3 | β-Sitostanol | CO₂ | 200 | 85 | 180 | Gelatine | 75-130 |
| 4 | β-Sitostenyllaurat | CO₂ | 200 | 85 | 175 | Chitosan | 55-140 |
| 5 | β-Sitostanylstearat | CO₂ | 200 | 85 | 175 | Gelatine/Chitosan (1:1) | 60-150 |
| 6 | Phytosterol* | - | - | - | - | - Gelatine/Chitosan (1:1) | 65-150 |

| | | | | | | | |
|---|---|---|---|---|---|---|---|
| *) 58,1 Gew.-% β-Sitosterol, 29,8 Gew.-% Campesterol, 4,5 Gew.-% Stigmasterol; 3,8 Gew.-% Tocopherol; 0,4 Gew.-% Cholesterol; 0,3 Gew.-% Squalan; ad 100 Unverseifbares. | | | | | | | |

**Anwendungsbeispiele.** Es wurden Gelatinekapseln (Gewicht ca. 1,5 g) mit einem Gehalt von 5 Gew.-% β-Sitostanol bzw. β-Sitostanolester (Nanopartikel eingeschlossen in eine Gelatine- bzw. Chitosanmatrix bzw. nichtnanoskalige Handelsprodukte) sowie 0,5 Gew.-% radioaktiv markiertem Cholesterin hergestellt. Zur Untersuchung der hypocholesterinämischen Wirkung ließ man männliche Ratten (Einzelgewicht ca. 200 g) über Nacht fasten. Am folgenden Tag wurde den Versuchstieren jeweils eine zerkleinerte Gelatinekapsel mit etwas kochsalzhaltigem Wasser über eine Magensonde eingeführt. Nach 3, 6, 12, 24 und 48 h wurde den Tieren Blut abgenommen und der Gehalt an radioaktivem Cholesterin bestimmt. Die Ergebnisse, die den Mittelwert der Messungen von 10 Versuchstieren darstellen, sind in Tabelle 2 zusammengefaßt. Die Angaben zur Abnahme der Radioaktivität verstehen sich jeweils in Bezug auf eine Blindgruppe von Versuchstieren, denen lediglich Gelatinekapseln mit einem Gehalt von 20 Gew.-% Vitamin E und einer entsprechenden Menge radioaktiv markiertem Cholesterin verabreicht worden war. Die Beispiele 1 und 2 sind erfindungsgemäß, die Beispiele V1 und V2 dienen dem Vergleich.

**Tabelle 2**

| **Hypocholesterinämische Wirkung** | | | | | | |
|---|---|---|---|---|---|---|
| **Bsp.** | **Phytosterol(ester)** | **Radioaktivität [%-rel.]** | | | | |
| | | **nach 3 h** | **nach 6 h** | **nach 12 h** | **nach 24 h** | **nach 48 h** |
| V1 | β-Sitostanol* | 93 | 83 | 75 | 50 | 32 |
| V2 | β-Sitostanylstearat* | 90 | 80 | 71 | 44 | 26 |
| 1 | Nano-β-Sitostanol** | 88 | 77 | 69 | 44 | 27 |
| 2 | Nano-β-Sitostanylstearat*** | 85 | 74 | 66 | 37 | 21 |

| | | | | | | |
|---|---|---|---|---|---|---|
| *) Handelsprodukte | | | | | | |
| **) gem. Herstellbeispiel 3 | | | | | | |
| ***) gem. Herstellbeispiel 4 | | | | | | |

## Patentansprüche

1. Verwendung von nanoskaligen Sterolen und/oder Sterolestem, welche von einem toxikologisch unbedenklichen Schutzkolloid ummantelt vorliegen, mit Teilchendurchmessern im Bereich von 10 bis 300 nm als Lebensmittelzusatzstoffe.

2. Verwendung nach Anspruch 1, **dadurch gekennzeichnet, dass** man Phytosterole oder deren Ester einsetzt.

3. Verwendung nach den Ansprüchen 1 und 2, **dadurch gekennzeichnet, dass** man Sitosterole oder deren Ester einsetzt.

4. Verwendung nach mindestens einem der Ansprüche 1 bis 3, **dadurch gekennzeichnet, dass** man nanoskalige Sterole und/oder Sterolester einsetzt, die man erhält, indem man
(a) die Ausgangsstoffe unter überkritischen oder nahekritischen Bedingungen in einem geeigneten Lösungsmittel löst,
(b) die fluide Mischung über eine Düse in ein Vakuum, ein Gas oder eine Flüssigkeit entspannt, und
(c) das Lösemittel dabei gleichzeitig verdampft.

5. Verwendung nach Anspruch 4, **dadurch gekennzeichnet, dass** man als Schutzkolloid Gelatine und/oder Chitosan einsetzt.

6. Verwendung nach mindestens einem der Ansprüche 1 bis 5, **dadurch gekennzeichnet, dass** man die Sterole und/oder Sterolester in Mengen von 0,01 bis 5 Gew.-% - bezogen auf die Mittel - einsetzt.

7. Verwendung nach mindestens einem der Ansprüche 1 bis 6, **dadurch gekennzeichnet, dass** man die Sterole und/oder Sterolester in Butter, Margarine, Diätnahrung, Fritierölen, Speiseölen, Mayonnaisen, Salatdressings, Kakaoprodukten oder Wurst einsetzt.

8. Verwendung von nanoskaligen Sterolen und/oder Sterolestern, welche von einem toxikologisch unbedenklichen Schutzkolloid ummantelt vorliegen, mit Teilchendurchmessem im Bereich von 10 bis 300 nm als Wirkstoff zur Herstellung von hypocholesterinämischen Mitteln.

## Claims

1. The use of nanoscale sterols and/or sterol esters with particle diameters of 10 to 300 nm surrounded by a toxicologically safe protective colloid as food additives.

2. The use claimed in claim 1, **characterized in that** phytosterols or their esters are used.

3. The use claimed in claims 1 and 2, **characterized in that** sitosterols or their esters are used.

4. The use claimed in at least one of claims 1 to 3, **characterized in that** nanoscale sterols and/or sterol esters obtained by
(a) dissolving the starting materials in a suitable solvent under supercritical or near-critical conditions,
(b) expanding the fluid mixture through a nozzle into a vacuum, a gas or a liquid and
(c) simultaneously evaporating the solvent
are used.

5. The use claimed in claim 4, **characterized in that** gelatine and/or chitosan is/are used as the protective colloid.

6. The use claimed in at least one of claims 1 to 5, **characterized in that** the sterols and/or sterol esters are used in quantities of 0.1 to 5% by weight, based on the food.

7. The use claimed in at least one of claims 1 to 6, **characterized in that** the sterols and/or sterol esters are used in butter, margarine, diet food, frying oils, edible oils, mayonnaises, salad dressings, cocoa products or sausage.

8. The use of nanoscale sterols and/or sterol esters with particle diameters of 10 to 300 nm surrounded by a toxicologically safe protective colloid as active substances for the production of hypocholesterolemic agents.

## Revendications

1. Utilisation de stérols et/ou d'esters de stérols à l'échelle nanométrique, qui se présentent sous forme enrobée dans un colloïde protecteur dépourvu de nocivité toxicologique, et avec des diamètres particulaires situés dans un intervalle allant de 10 à 300 nm, comme additifs pour produits alimentaires.

2. Utilisation selon la revendication 1,
**caractérisée en ce qu'**
on utilise des phytostérols ou leurs esters.

3. Utilisation selon les revendications 1 et 2,
**caractérisée en ce qu'**
on utilise des sitostérols ou leurs esters.

4. Utilisation selon au moins une des revendications 1 à 3,
**caractérisée en ce qu'**
on utilise des stérols et/ou des esters de stérols à l'échelle nanométrique, que l'on obtient
a) en dissolvant les produits de départ dans un solvant approprié dans des conditions hypercritiques ou presque critiques,
b) en détendant le mélange fluide au moyen d'une buse dans le vide, un gaz ou un liquide, et
c) en vaporisant simultanément le solvant dans cette opération.

5. Utilisation selon la revendication 4,
**caractérisée en ce que**
comme colloïde protecteur on utilise des gélatines et/ou du chitosane.

6. Utilisation selon au moins une des revendications 1 à 5,
**caractérisée en ce qu'**
on utilise les stérols et/ou les esters de stérols en quantités de 0,01 à 5 % en poids - par rapport aux produits.

7. Utilisation selon au moins une des revendications 1 à 6,
**caractérisée en ce qu'**
on utilise les stérols et/ou les esters de stérols dans du beurre, de la margarine, des aliments diététiques, des huiles de friture, des huiles alimentaires, des mayonnaises, des vinaigrettes pour salades, des produits de cacao ou de la charcuterie.

8. Utilisation de stérols et/ou d'esters de stérols à l'échelle nanométrique, qui se présentent sous forme enrobée dans un colloïde protecteur dépourvu de nocivité toxicologique, et ayant des diamètres particulaires allant de 10 à 300 nm, comme substances actives pour la préparation de produits hypocholestérolémiques.
